# EUROPÄISCHE PATENTSCHRIFT

(11) **EP 1 205 204 B1**
(45) Veröffentlichungstag und Bekanntmachung des Hinweises auf die Patenterteilung: **03.10.2007**
(21) Anmeldenummer: 00124103.3
(22) Anmeldetag: 07.11.2000
(51) Int. Cl.: A61M 16/06

(54) **Verfahren zur Vorortherstellung einer Beatmungsmasken**
Method of fabricating on site a breathing mask
Méthode de fabrication sur place d'un masque individuel

(43) Veröffentlichungstag der Anmeldung: 15.05.2002
(73) Patentinhaber: Velten, Andreas, 39307 Genthin (DE)
(72) Erfinder: Velten, Andreas, 39307 Genthin (DE); Schilling, Norbert, 97277 Neubrunn (DE)
(74) Vertreter: Heyner, Klaus

(56) Entgegenhaltungen:
- WO-A-98/48878
- WO-A-99/58181
- WO-A-99/58198
- DE-A- 19 914 667
- DE-C- 28 036
- FR-A- 2 658 725
- US-A- 4 782 832

## Beschreibung

Die Erfindung betrifft eine Vorrichtung (Connector) und ein Verfahren, welches der einfachen und schnellen Herstellung von individuellen Beatmungsmasken für die Intensiv-, Klinik- und Heimbeatmung sowie der CPAP und nCPAP-Therapie (nasal Continous-Positive-Airway-Pressure-Therapy) und anderer Therapieformen für die nasale Beatmung dient.

Konfektionierte und laborgefertigte individuelle Beatmungsmasken weisen nicht unerhebliche Nachteile auf. So sind häufig Undichtigkeiten zu beobachten, die insbesondere bei der nasalen CPAP-Therapie, eine überwiegend bei der Therapie des obstruktiven Schlafapnoe-Syndroms angewendete Behandlungsform, auftreten. Infolge der Undichtigkeiten wird oftmals eine Beatmungsdruckerhöhung veranlasst, um eine zielgerichtete Beatmung durchführen zu können. Außerdem wird zur Vermeidung der Undichtigkeiten der Zug an den Haltevorrichtungen der Masken erhöht, was wiederum zur vermehrten Bildung von Druckstellen, insbesondere auf dem Nasenrücken, an der Nasenscheidewand oder der Nasenwurzel, führt. Ein weiterer Nachteil ergibt sich aus der Größe der Masken, was, verbunden mit psychische Beschwerden, zu einem eingeschränkten Tragekomfort führt. Bei laborgefertigten individuellen Masken besteht außerdem wegen dem hohen Aufwand bei ihrer Herstellung das Problem in der schnellen Verfügbarkeit

Aus dem Stand der Technik ist die US 4 782 832 A vorbekannt, in der eine Beatmungsmaske, bestehend aus einem Träger mit einem Geräteanschluss sowie zwei auf dem Träger platzierte nasale Endstücke, offenbart ist. Die nasalen Endstücke umfassen bei einer ersten Ausgestaltung jeweils einen Exzenter, einen zylinderförmigen Aufsatz mit nichtparalleler Grund- und Deckfläche sowie einen Dichtkonus. Mittels des Exzenters und des Aufsatzes erfolgt die an die Anatomie der Nase angepasste Verstellung der nasalen Endstücke. Bei einer zweiten Ausgestaltung der Erfindung sind anstelle des Exzenters und des Aufsatzes zwei orthogonal zueinander angeordnete Faltenbalge vorgesehen, die zur allseitigen Verstellung der nasalen Endstücke eingesetzt werden. Eine dritte Ausgestaltung der Beatmungsmaske weist eine sackförmig ausgebildete starre Hülle auf, an deren Rückseite eine rohrförmiger Geräteanschluss angeordnet ist und von deren Vorderseite sich zwei nichtverstellbare Dichtkonus erstrecken. Nachteilig an dieser Erfindung ist die werkseitige Vorfertigung der Beatmungsmaske, welche nur bedingt die unterschiedlichen anatomischen Gegebenheiten der Träger berücksichtigt. Außerdem ist die Beatmungsmaske sehr aufwendig gefertigt und besteht aus vielen Einzelkomponenten.

Der FR 2 658 725 A ist eine Nasenmaske mit Dichtlippe zu entnehmen, welche durch ein Thermoforming-Verfahren individuell gefertigt wird. Die Herstellung erfolgt hierbei unter Verwendung einer Matrize beim Orthopäden. Dazu wird unter Verwendung eines Formsands ein Nasenabdruck des Trägers genommen und anschließend eine Negativform zur Herstellung eines starren Maskenkörpers erstellt. Der Maskenkörper seinerseits entspricht nunmehr der Anatomie des Nasenbereichs des Trägers und ergänzt sich gemeinsam mit zwei rohrförmigen Tuben zur Nasenmaske. Nachteilig bei dieser Erfindung ist zum einen, dass diese Beatmungsmaske nur sehr aufwendig beim Orthopäden oder von einem Arzt gefertigt werden kann, und zum anderen, dass der angefertigte Maskenkörper starr ist, was dazu führt, dass ein dauerhafter optimaler Passsitz nicht gewährleistet ist.

In der WO 99/58181 A ist eine nasale Beatmungsmaske beschrieben, die ein stabilisierendes Mundstück sowie daran federn gelagerte verstellbare nasale Endstücke aufweist. Das Mundstück greift hierbei in die obere Zahnreihe des Trägers ein. An Stelle der nasalen Endstücke kann auch ein Maskenkörper oder eine Kombination aus Maskenkörper und nasale Endstücke vorgesehen sein. Die nasalen Endstücke und der Maskenkörper können hierbei eine verschiedene Größe und Form aufweisen. Als besonderer Nachteil dis Erfindung ist die Tatsache zu nennen, dass eine Reinigung der Beatmungsmaske wegen der unterschiedlichen eingenommenen Räume der Beatmungsmaske (Nasenraum und Mundraum) recht aufwendig. Außerdem kann wegen der vorgefertigten Formgebung keine vollständige Abdichtung der Beatmungsmaske an ihrem Träger erreicht werden.

Die Aufgabe der Erfindung besteht nunmehr darin, ein Verfahren zur einfachen und individuellen Vorortherstellung einer Beatmungsmaske vorzuschlagen, welche an die anatomischen Gegebenheiten ihres Trägers angepasst ist und auch bei längerer Benutzung Undichtigkeiten und Druckstellen zuverlässig verhindert.

Diese Aufgabe wird durch ein Verfahren zur Vorortherstellung einer Beatmungsmaske unter Verwendung einer Verbindungsvorrichtung gelöst, wobei die Verbindungsvorrichtung hierzu zumindest einen Geräteanschluss sowie mindestens ein, vorzugsweise zwei mit einem Durchlass für Atemgas versehene einkürzbare und im Nasenloch platzierbare nasale Endstücke aufweist.

Zur Vorortherstellung der Beatmungsmaske sind erfindungsgemäß folgende Verfahrensschritte vorgesehen:
(a) Einkürzen der nasalen Endstücke, insbesondere Zapfen, der Verbindungsvorrichtung in Abhängigkeit der Größe der Nasenlöcher des Trägers bis ein einwandfreier spannungsloser Sitz gewährleistet ist,
(b) Einführen der nasalen Endstücke, insbesondere Zapfen, der Verbindungsvorrichtung in die Nasenlöcher,
(c) Verteilen eines medizinischen Silikon-Elastomers um die Verbindungsvorrichtung und um den Nasenbereich, solange sich das Silikon-Elastomer noch in einer plastischen Phase befindet.

Die mit diesem Verfahren erhältlichen Beatmungsmasken werden für die nasale Beatmung einer Person eingesetzt. Dabei wird die Beatmungsmaske aus einer aus weichbleibenden medizinischen Silikon-Elastomer (medical grade 2) gefertigten Verbindungsvorrichtung (Connector), an der zugleich zumindest ein als bewegliches Luft/Sauerstoff-Anschlussstück ausgebildeter Geräteanschluss platziert ist, und einem ebenso weichbleibenden medical grade 2-Silikon, durch Umspritzen der nasalen Anteile des Gesichts, insbesondere der Nasenlöcher und der Nasenspitze, direkt am Patienten bzw. Träger erstellt. Die Befestigungselemente für die Aufnahme der Halterung werden direkt in diesen Kunststoff eingearbeitet.

Die Abdichtung der Verbindungsvorrichtung (Connector) erfolgt im Bereich der Nasenlöcher primär durch die vorgeformte Art der nasalen Endstücke (Zapfen) der Verbindungsvorrichtung und sekundär durch das Verteilen des Silikon-Elastomers.

Die Verteilung des vorzugsweise aus Silikon ausgebildeten Kunststoffs der Härte 10 bis 70 (Shore A) um die Verbindungsvorrichtung (Connector) erfolgt derart, dass sämtliche Zwischenräume zwischen den Nasenlöchern und der Verbindungsvorrichtung verschlossen sind, und die Nase in einer gleichmässigen Schicht vom Silikon umgeben ist. Solange sich das Silikon noch in der plastischen Phase befindet, wird das Befestigungselement platziert. Dies erfolgt in der Form, dass die Nasenspitze umgeben wird und das Silikonmaterial nicht verdrängt wird. Zur Stabilisierung wird nachfolgend eine zweite Schicht des Materials über die Randbereiche des Befestigungselementes appliziert. Eine Isolierung der Haut ist nicht erforderlich. Abschliessend werden, nach dem Abnehmen der fertigen Maske die Ränder mittels Schere und Skalpell versäubert und die Haltebänder oder das Kopfgeschirr angebracht. Diese individuelle Atemmaske kann mit einem Kopfband aus Neopren, Schaumstoff oder Dreipunktkopfband oder entsprechenden konfektionierten Halteelementen befestigt werden.

Durch die enorme Verkleinerung der Maske wird der Luft-Totraum in der Maske erheblich gegenüber konventionellen Masken reduziert. Infolge des direkten Einströmens der Luft ist eine Reduzierung des Beatmungsdruckes, welcher in der Regel bei 3-25 mbar liegt, angezeigt.

Weitere Merkmale der Erfindung ergeben sich aus den Ansprüchen und aus der nachfolgenden Beschreibung, in der ein Anführungsbeispiel einer erfindungsgemässen Beatmungsmaske mit entsprechenden Verweisen auf die beigefügten Abbildungen.

Dabei zeigt:

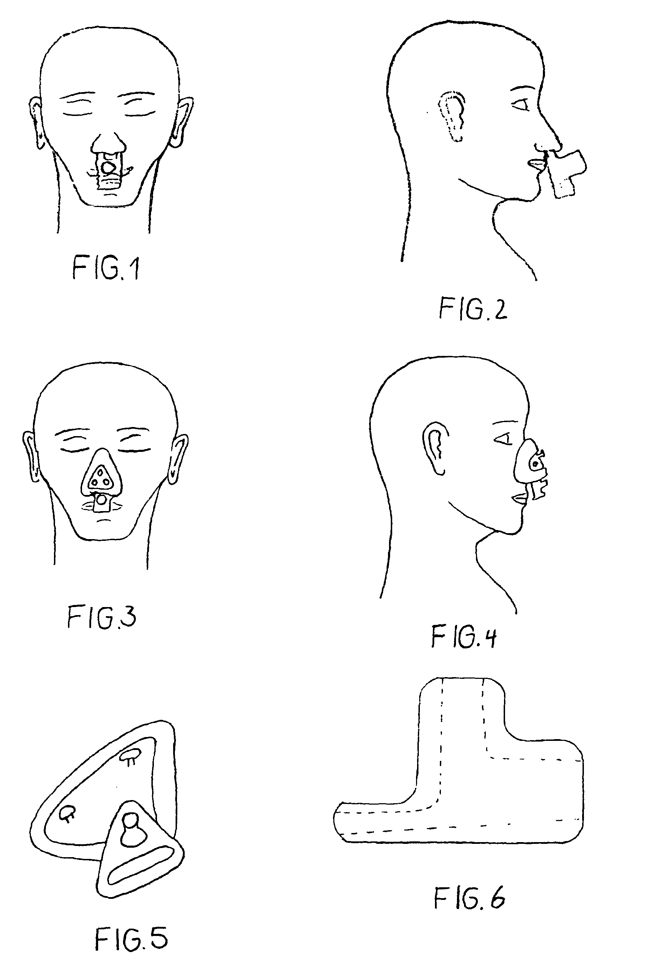

**Fig. 1**. die Frontalansicht einer Person mit einer erfindungsgemässen Verbindungsvorrichtung, welche von der Person getragen wird.
**Fig. 2**. die Seitenansicht einer Person mit einer erfindungsgemässen Verbindungsvorrichtung, welche von der Person getragen wird.
**Fig. 3.** die Frontalansicht der erfindungsgemässen Beatmungsmaske in Detailansicht,welche von der Person getragen wird.
**Fig. 4.** die Seitenansicht der erfindungsgemässen Beatmungsmaske in Detailansicht, welche von der Person getragen wird.
**FIG. 5.** das Befestigungselement der erfindungsgemässen Beatmungsmaske in Detailansicht
**Fig. 6**. Detailansicht der erfindungsgemässen Verbindungsvorrichtung (Connector)

## Patentansprüche

1. Verfahren zur Vorortherstellung einer Beatmungsmaske unter Verwendung einer Verbindungsvorrichtung, die zumindest einen Geräteanschluss sowie mindestens ein, vorzugsweise zwei mit einem Durchlass für Atemgas versehene einkürzbare und im Nasenloch platzierbare nasale Endstücke aufweist, umfassend folgende Verfahrensschritte:
(a) Einkürzen der nasalen Endstücke, insbesondere Zapfen, der Verbindungsvorrichtung in Abhängigkeit der Größe der Nasenlöcher des Trägers bis ein einwandfreier spannungsloser Sitz gewährleistet ist,
(b) Einführen der nasalen Endstücke, insbesondere Zapfen, der Verbindungsvorrichtung in die Nasenlöcher,
(c) Verteilen eines medizinischen Silikon-Elastomers um die Verbindungsvorrichtung und um den Nasenbereich, solange sich das Silikon-Elastomer noch in einer plastischen Phase befindet.

2. Verfahren nach Anspruch 1, **dadurch gekennzeichnet, dass** die Abdichtung der Verbindungsvorrichtung im Bereich der Nasenlöcher primär durch die vorgeformte Art der nasalen Endstücke, insbesondere Zapfen, der Verbindungsvorrichtung und sekundär durch das Verteilen des Silikon-Elastomers erfolgt.

## Claims

1. Method of fabricating on site a breathing mask by using a connector, which is provided with an equipment connection port and at least one, preferably two shortenable nasal end pieces placeable in the nostril, provided with a passage for respiratory gas, comprising the following process steps:
(a) shortening of the nasal end pieces, particularly plugs, of the connector depending on the dimensions of the carrier's nostrils until perfect stress-free fit is ensured;
(b) inserting of the nasal end pieces, particularly plugs, of the connector into the nostrils;
(c) distributing of a medical silicone elastomer around the connector and the nose region while the silicone elastomer is in a plastic phase.

2. Method to claim 1 **characterized in that** the sealness of the connector in the region of the nostrils is, primarily, based on the preformed design of the nasal end pieces, particularly plugs, of the connector and, secondarily, on the distributing of the silicone elastomer.

## Revendications

1. Procédé de fabrication sur place d'un masque respiratoire en utilisant un dispositif de connexion qui présente au moins un raccordement d'appareil ainsi qu'un, de préférence deux embouts nasaux munis d'un passage pour du gaz respirable, raccourcissables et pouvant être placés dans les narines, comprenant les étapes de procédé suivantes :
a) raccourcissement des embouts nasaux, en particulier tenons, du dispositif de connexion en fonction de la grandeur des narines du porteur jusqu'à ce qu'un positionnement correct et sans contrainte soit assuré,
b) introduction des embouts nasaux, en particulier tenons, du dispositif de connexion dans les narines,
c) répartition d'un élastomère silicone médical autour du dispositif de connexion et autour de la région nasale tant que l'élastomère silicone se trouve encore en phase plastique.

2. Procédé selon la revendication 1, **caractérisé en ce que** l'étanchéité du dispositif de connexion dans la région des narines est assurée en premier lieu par la nature préformée des embouts nasaux, en particulier tenons, du dispositif de connexion et en deuxième lieu par la répartition de l'élastomère silicone.
